# EUROPÄISCHE PATENTSCHRIFT

(11) **EP 4 281 395 B1**
(45) Veröffentlichungstag und Bekanntmachung des Hinweises auf die Patenterteilung: **10.09.2025**
(21) Anmeldenummer: 22701233.3
(22) Anmeldetag: 20.01.2022
(51) Int. Cl.: B65G 47/14, B65G 47/19, B65G 47/20, B67B 3/064, A61L 2/26, B01L 1/02, B65G 65/23

(54) **SYSTEM ZUM TRANSPORT VON STERILEN SCHÜTTFÄHIGEN VERSCHLUSSELEMENTEN**
SYSTEM FOR TRANSPORTING LOOSE STERILE CLOSURE ELEMENTS
SYSTÈME POUR TRANSPORTER DES ÉLÉMENTS DE FERMETURE STÉRILES EN VRAC

(30) Priorität: 22.01.2021 DE 102021101384
(43) Veröffentlichungstag der Anmeldung: 29.11.2023
(73) Patentinhaber: Syntegon Technology GmbH, 71332 Waiblingen (DE)
(72) Erfinder: NAGLER, Stefan, 73485 Unterschneidheim (DE); KRAUSS, Ulrich, 74532 Ilshofen (DE); ILGENFRITZ, Markus, 91555 Feuchtwangen (DE); KÜHNLE, Albrecht, 74564 Crailsheim (DE)
(74) Vertreter: Novagraaf Group
(86) Internationale Anmeldenummer: PCT/EP2022/051178
(87) Internationale Veröffentlichungsnummer: WO 2022/157219

(56) Entgegenhaltungen:
- WO-A2-2004/042381
- AU-B2- 491 985
- FR-A1- 2 866 016
- US-A- 2 696 285
- US-A- 3 086 639

## Beschreibung

Die Erfindung betrifft ein aus der EP 3 613 442 A1 bekanntes System zum Transport von sterilen schüttfähigen Verschlusselementen aus einer Umgebung eines Isolators in einen Innenraum des Isolators, umfassend einen Behälter zur Speicherung einer Vorrats-Menge von Verschlusselementen in der Umgebung des Isolators, eine Isolatoröffnung und eine Sammeleinrichtung zum Sammeln der Verschlusselemente und zur Bereitstellung der Verschlusselemente in dem Innenraum des Isolators. Aus der FR 2 866 016 A1 ist ein System mit den Merkmalen des Oberbegriffs des Patentanspruchs 1 bekannt. Aus der US 2 696 285 A, der AU 491 985 B2, der US 3 086 639 A und der WO 2004/042381 A2 sind weitere Transportsysteme bekannt.

Der innerhalb eines Isolators zur Verfügung stehende Raum ist prinzipbedingt begrenzt. Es ist daher erstrebenswert, diesen Raum möglichst produktiv zu nutzen, also insbesondere für die Handhabung eines Verschlusselements und dessen Positionierung an oder auf einem innerhalb des Isolators angeordneten, befüllten und zu verschließenden Behältnis.

Der vorliegenden Aufgabe liegt die Aufgabe zugrunde, ein System zum Transport von sterilen schüttfähigen Verschlusselementen anzugeben, das eine möglichst produktive Nutzung des Innenraums des Isolators ermöglicht.

Diese Aufgabe wird gelöst durch ein System mit den Merkmalen des Patentanspruchs 1.

Erfindungsgemäß kann die Bevorratung der Verschlusselemente im Wesentlichen außerhalb des Isolators erfolgen, während der innerhalb des Isolators angeordneten Sammeleinrichtung nur eine vergleichsweise kleinere Soll-Menge von Verschlusselementen zugeführt wird. Die Dosierung der Verschlusselemente ermöglicht es, den Innenraum des Isolators weitestgehend produktiv zu nutzen, also beispielsweise Behältnisse mit Verschlusselementen zu verschließen. Die Dosiervorrichtung sichert einen zuverlässigen Nachschub für die produktive Nutzung des Innenraums des Isolators, insbesondere autark und unabhängig von Bedienpersonal.

Die transportierende Soll-Menge ist durch eine absolute Menge von Verschlusselementen definiert (bspw. "20 Stück") oder durch eine auf eine zeitliche Einheit bezogene absolute Menge von Verschlusselementen (bspw. "20 Stück pro Minute" oder "1 Stück alle 3 Sekunden").

Eine bevorzugte Ausgestaltung sieht vor, dass die Dosiervorrichtung mindestens einen Aktor aufweist, der in Abhängigkeit der zu transportierenden Soll-Menge zwischen einem Ruhezustand und einem Gebrauchszustand schaltbar ist. Dabei ist es denkbar, dass der Aktor während des produktiven Betriebs des Isolators dauerhaft aktiv ist und Verschlusselemente mit einer vorgebbaren Frequenz (Menge pro Zeit) aus dem Behälter in die Sammeleinrichtung transportiert werden. Es ist aber auch denkbar, dass der Aktor intermittierend in einen Gebrauchszustand versetzt wird und Verschlusselemente chargenweise transportiert werden, beispielsweise mit Zuführung einer Soll-Menge von X Verschlusselementen, sich anschließender Unterbrechung und sich danach anschließender Zuführung einer weiteren Soll-Menge von X oder Y Verschlusselementen.

Der Gebrauchszustand des Aktors geht vorzugsweise mit einer Erhöhung der potentiellen und/oder kinetischen Energie zumindest einer Teilmenge der Vorrats-Menge der Verschlusselemente einher. Es ist also möglich, die gesamte Vorrats-Menge des Behälters in einen höheren Energiezustand zu versetzen oder aber nur eine Teilmenge der Vorrats-Menge.

Vorzugsweise ist eine in der Umgebung, also außerhalb des Isolators angeordnete Auflage für den Behälter vorgesehen, wobei der mindestens eine Aktor die Neigung und/oder einen Vibrationszustand der Auflage und des Behälters verändert. Beispielsweise kann ein Behälter ausgehend aus einer flacheren Raumlage stärker geneigt werden, sodass die innerhalb des Behälters angeordnete Verschlusselemente bezogen auf die Schwerkraftrichtung weiter nach oben verlagert werden und von dort der Schwerkraftrichtung folgend in Richtung nach unten fallen oder rutschen können. In entsprechender Weise ist es möglich, einen Behälter mit Verschlusselementen, ausgehend aus einer stärker geneigten Lage, wieder in eine flachere Lage zu verbringen, um die Tendenz der Verschlusselemente, nach unten zu fallen oder zu rutschen, zu hemmen oder zu stoppen.

Es ist auch möglich, dass die Auflage mit einer Vibrationseinrichtung zusammenwirkt, welche den Behälter und die darin bevorrateten Verschlusselemente in einen Vibrationszustand versetzt. Dieser Vibrationszustand kann eine in Richtung Behälteröffnung gerichtete Bewegungskomponente aufweisen, sodass die Verschlusselemente angeregt sind, den Behälter zu verlassen und in Transportrichtung durch die Isolatoröffnung hindurch in die Sammeleinrichtung transportiert zu werden.

Der Behälter ist als Beutel oder als Container ausgebildet. Diese Behälter dienen zum Transport von bereits sterilisierten Verschlusselementen und können in der Umgebung eines Isolators zur Verfügung gestellt werden. Es ist bekannt, solche Behälter an sogenannten RTP (Rapid Transfer Ports) eines Isolators anzudocken, wobei im Bereich der Isolatoröffnung eine Doppeltür verwendet wird. Ein erster Teil einer solchen Doppeltür ist dem Isolator zugeordnet (als "alpha-Teil"). Ein zweiter Teil einer solchen Doppeltür ist durch einen Behälterverschluss / einen Deckel des Behälters bereitgestellt (als "beta-Teil"), wobei die beiden genannten Teile der Doppeltür miteinander mechanisch koppelbar sind, sodass diese beiden Teile gemeinsam eine Doppeltür bilden, welche in einem geöffneten Zustand eine Isolatoröffnung freigibt, durch welche hindurch Verschlusselemente aus dem Behälter in den Innenraum des Isolators transportiert werden können.

Die Dosiervorrichtung umfasst mindestens eine Fördereinheit, mit einer Förderkammer, deren Volumen größer ist als das Volumen eines einzelnen Verschlusselements und kleiner als das Volumen von zwei Verschlusselementen. Eine solche Förderkammer ermöglicht eine vereinzelte Dosierung von Verschlusselementen. Für einen besonders schonenden Transport der Verschlusselemente ist es bevorzugt, wenn sich die Förderkammer - entlang einer Transportrichtung der Verschlusselemente gesehen - in ihrem Volumen erweitert.

Bei einer bevorzugten Ausgestaltung einer Fördereinheit ist eine Fördertrommel vorgesehen, welche eine - bezogen auf eine Transportachse der Verschlusselemente - äußere Begrenzung der Förderkammer bildet. Eines solche Fördertrommel umschließt also zumindest einen Teil des Transportwegs der Verschlusselemente.

Es ist möglich, dass die Fördertrommel zumindest einen Fördertrommelabschnitt aufweist, der auf Höhe des Isolatoröffnung anordenbar oder angeordnet ist und eine Begrenzung der Isolatoröffnung überdeckt, dass zu transportierende Verschlusselemente nicht in Kontakt mit der Begrenzung der Isolatoröffnung gelangen können.

Für einen schonenden und einfachen Transport der Verschlusselemente ist es ferner bevorzugt, wenn die Fördertrommel, bezogen auf eine Transportachse der Verschlusselemente, einen hohlzylindrischen Abschnitt aufweist und/oder wenn sich die Fördertrommel in Transportrichtung der Verschlusselemente gesehen hohlkegelförmig erweitert.

Ferner ist es bevorzugt, wenn die Fördereinheit mindestens ein Förderelement aufweist, das die Förderkammer entlang einer Transportachse der Verschlusselemente gesehen begrenzt. Das mindestens eine Förderelement dient zur Vereinzelung einer Mehrzahl von Verschlusselementen. Ein Förderelement kann beispielsweise in Form eines Leitstegs, einer Förderspirale oder -schnecke oder eines Förderrads ausgebildet sein.

Es ist ferner bevorzugt, wenn die Dosiervorrichtung eine in Abhängigkeit der zu transportierenden Soll-Menge schaltbare Förderantriebseinrichtung zum Drehantrieb der Fördertrommel und/oder der Förderelemente umfasst. Diese Förderantriebseinrichtung kann kontinuierlich oder intermittierend betrieben werden.

Es ist ferner möglich, dass die Dosiereinrichtung eine Behälterantriebseinrichtung zur Drehung des Behälters oder eines Teilabschnitts des Behälters um eine zentrale Behälterachse umfasst. Auch auf diese Weise kann der Bewegungszustand der Verschlusselemente innerhalb des Behälters oder innerhalb eines Teilabschnitts des Behälters angeregt werden. Diese Anregung kann außerhalb des Isolators erfolgen oder aber im Bereich eines innerhalb des Isolators angeordneten Teilabschnitts des Behälters.

Vorzugsweise weist ein innerhalb des Isolators angeordneter Teilabschnitt des Behälters eine Austrittsöffnung auf, deren Querschnitt so bemessen ist, dass maximal ein Verschlusselement gleichzeitig durch diese Austrittsöffnung hindurch transportiert werden kann.

Es ist möglich, dass die Dosiereinrichtung eine Verschlusselementleitung umfasst, welche einen Überbrückungsabschnitt aufweist, der innerhalb der Isolatoröffnung anordenbar oder angeordnet ist. Der Überbrückungsabschnitt überdeckt eine Begrenzung der Isolatoröffnung, sodass zu transportierende Verschlusselemente nicht in Kontakt mit der Begrenzung der Isolatoröffnung gelangen können. Eine Verschlusselementleitung kann durch eine starre Leitung gebildet sein (beispielsweise durch ein Rohr) oder durch eine biegeschlaffe Leitung (beispielsweise durch einen Schlauch).

Eine besonders bevorzugte Ausführungsform sieht vor, dass die Dosiereinrichtung eine Zähleinrichtung zur Erfassung einer Anzahl, eines Gewichts und/oder eines Volumens der Verschlusselemente umfasst, wobei vorzugsweise die Zähleinrichtung in Transportrichtung der Verschlusselemente gesehen vor der Sammeleinrichtung angeordnet ist. Eine solche Zähleinrichtung ermöglicht die Bildung eines Regelsystems und die Erfassung einer Ist-Menge von Verschlusselementen, die mit einer vorgegebenen Soll-Menge von Verschlusselementen verglichen werden kann. Ein vorstehend beschriebener Aktor und/oder eine Förderantriebseinrichtung und/oder eine Behälterantriebseinrichtung kann oder können dann in Abhängigkeit einer Differenz zwischen der vorgegebenen Soll-Menge und der erfassten Ist-Menge von Verschlusselementen angesteuert werden.

Zur weiteren Erhöhung der Produktivität ist es bevorzugt, wenn die Dosiervorrichtung oder Teile der Dosiervorrichtung als innerhalb des Isolators bewegbare Baugruppe bereitgestellt ist oder sind, welche vorzugsweise mittels eines Roboterarms positionierbar ist. Die Baugruppe umfasst beispielsweise eines oder mehrere der nachfolgend genannten Bauteile, deren Funktion bereits vorstehend unter Bezugnahme auf die Unteransprüche 5 bis 11 beschrieben wurde. Dies sind im Einzelnen: Die Fördereinheit (mit einer Fördertrommel und/oder mindestens einem Förderelement) und/oder die Förderantriebseinrichtung und/oder die Behälterantriebseinrichtung (für den Fall, dass diese innerhalb des Isolators angeordnet ist) und/oder die Zähleinrichtung und/oder die Verschlusselementleitung.

Eine bevorzugte Ausgestaltung sieht vor, dass der Sammler als Sortiereinrichtung ausgebildet ist, wobei vorzugsweise der Sortiereinrichtung innerhalb des Isolators kein Zwischenspeicher für Verschlusselemente vorgeschaltet ist.

Insbesondere kann eine vergleichsweise kleine Sortiereinrichtung mit einer begrenzten Aufnahmekapazität (von beispielsweise maximal 50 Verschlusselementen oder von insbesondere maximal 20 Verschlusselementen) verwendet werden. Ein innerhalb des Isolators angeordneter Zwischenspeicher (auch "Bunker" genannt) ist nicht vorgesehen; der dadurch im Innenraum zusätzlich zur Verfügung stehenden Platz wird für die produzierenden Bauteile des Isolators genutzt.

Weitere Merkmale und Vorteile der Erfindung sind Gegenstand der nachfolgenden Beschreibung und der zeichnerischen Darstellung bevorzugter Ausführungsbeispiele.

In der Zeichnung zeigt
- Fig. 1: eine perspektivische Ansicht einer ersten Ausführungsform eines Systems zum Transport von sterilen schüttfähigen Verschlusselementen, wobei das System eine neigungsveränderbare Auflage für einen Behälter in Form eines Beutels umfasst;
- Fig. 2: eine perspektivische Ansicht einer weiteren Ausführungsform eines Systems zum Transport von sterilen schüttfähigen Verschlusselementen, wobei das System eine Vibrationsauflage für einen Behälter in Form eines Beutels umfasst;
- Fig. 3: eine perspektivische Ansicht einer weiteren Ausführungsform eines Systems zum Transport von sterilen schüttfähigen Verschlusselementen, wobei das System eine Vibrationsauflage für einen Behälter in Form eines Containers umfasst;
- Fig. 4 bis 6: perspektivische Ansichten einer weiteren Ausführungsform eines Systems zum Transport von sterilen schüttfähigen Verschlusselementen, wobei das System eine Fördereinrichtung mit einer hohlkegelförmigen Fördertrommel umfasst;
- Fig. 7 und 8: perspektivische Ansichten einer weiteren Ausführungsform eines Systems zum Transport von sterilen schüttfähigen Verschlusselementen, wobei das System eine Fördereinrichtung mit einer hohlzylindrischen Fördertrommel umfasst;
- Fig. 9 und 10: perspektivische Ansichten einer weiteren Ausführungsform eines Systems zum Transport von sterilen schüttfähigen Verschlusselementen, wobei das System eine Fördereinrichtung mit einem schneckenförmigen Förderelement umfasst, das gemeinsam mit einer Fördertrommel antreibbar ist;
- Fig. 11 und 12: perspektivische Ansichten einer weiteren Ausführungsform eines Systems zum Transport von sterilen schüttfähigen Verschlusselementen, wobei das System eine Fördereinrichtung mit einem schneckenförmigen Förderelement umfasst, das unabhängig von einer Fördertrommel antreibbar ist;
- Fig. 13 und 14: perspektivische Ansichten einer weiteren Ausführungsform eines Systems zum Transport von sterilen schüttfähigen Verschlusselementen, wobei das System eine Fördereinrichtung mit einem zellenradförmigen Förderelement umfasst; und
- Fig. 15: eine perspektivische Ansicht einer weiteren Ausführungsform eines Systems zum Transport von sterilen schüttfähigen Verschlusselementen, wobei das System einen Behälter in Form eines Containers umfasst, der einen drehbaren Teilabschnitt aufweist.

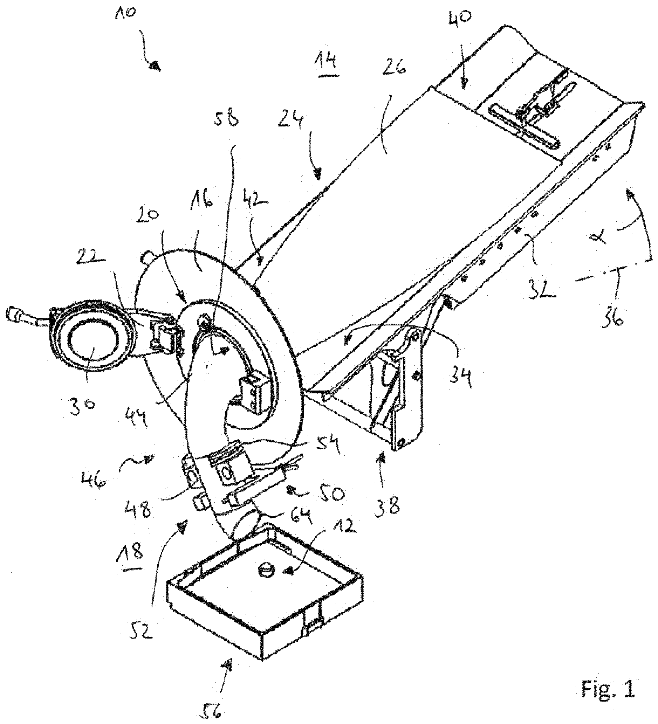

Ein System zum Transport von sterilen schüttfähigen Verschlusselementen ist in der Zeichnung insgesamt mit dem Bezugszeichen 10 bezeichnet. In Fig. 1 ist beispielhaft ein Verschlusselement 12 dargestellt, das beispielsweise als Kappe oder Stopfen ausgebildet sein kann und als Verschluss für ein zu verschließendes Behältnis (beispielweise einer Spritze) dient.

Das System 10 dient zum Transport solcher Verschlusselemente 12 aus einer Umgebung 14 eines Isolators 16 in einen Innenraums 18 des Isolators.

Der Isolator 16 ist nur abschnittsweise dargestellt und zwar im Bereich einer Isolatoröffnung 20, die mit einer Isolatortür 22 verschließbar ist.

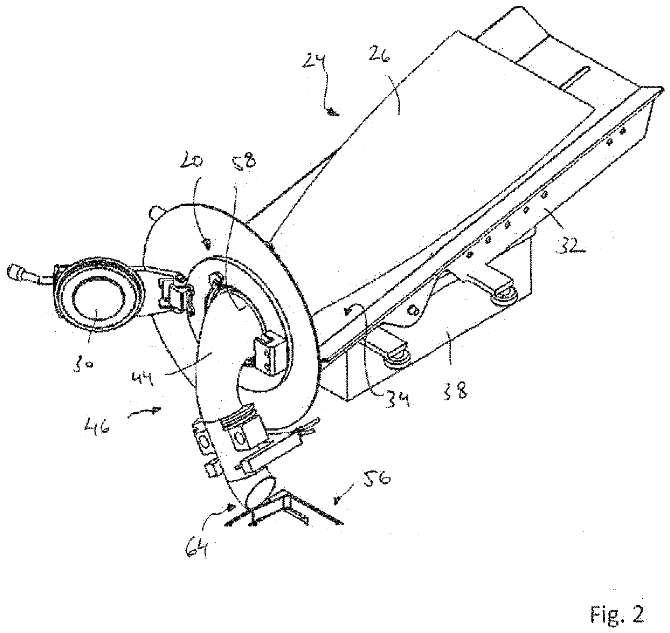

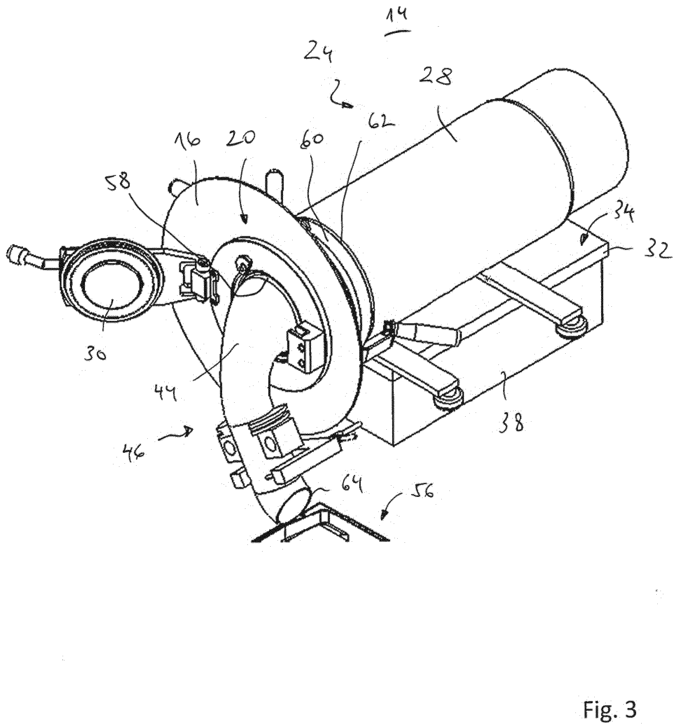

Zur Bevorratung von Verschlusselementen 12 in der Umgebung 14 des Isolators 16 ist ein Behälter 24 vorgesehen, der in Form eines Beutels 26 (vergleiche Fig. 1 und 2) oder eines Containers 28 (vergleiche Fig. 3) ausgebildet ist.

Unabhängig von der Ausgestaltung des Behälters 24 können solche Behälter 24 einen Behälterverschluss 30 aufweisen, der in einem Anlieferungszustand des Behälters 24 diesen verschließt. In einem solchen Anlieferungszustand verschließt die Isolatortür 22 die Isolatoröffnung 20 des Isolators 16.

Der Behälter 24 wird dann mit seinem Behälterverschluss 30 benachbart zu der Isolatortür 22 positioniert, sodass - zunächst noch in der Umgebung 14 des Isolators 16 - die Isolatortür 22 mechanisch mit dem Behälterverschluss 30 koppelbar ist. Der Behälter 24 weist einen den Behälterverschluss 30 ringförmig umfassenden Randabschnitt auf, der in der Umgebung 14 des Isolators 16 verbleibt. Bei einer Öffnung der Isolatortür 22 wird der Behälterverschluss 30 von dem Behälter 24 gelöst, und gemeinsam mit der Isolatortür 22 in den Innenraum 18 des Isolators 16 verbracht. Eine solche Konstruktion wird auch als "Doppeltür" eines "Rapid Transfer Ports" bezeichnet.

Das System 10 umfasst ferner eine in der Umgebung 14 des Isolators 16 angeordnete Auflage 32 mit einer Auflagefläche 34 zur Auflage eines Behälters 24.

Die Auflagefläche 34 kann sich - in einem Ausgangs- oder Ruhezustand - beispielsweise in einer horizontalen Ebene 36 erstrecken. Die Neigung der Auflage 32 und somit der Auflagefläche 34 entsprechend einem Neigungswinkel α ist mittels eines Aktors 38 einstellbar. Dabei korreliert eine größere Neigung mit einem größeren Höhenunterschied zwischen einem rückwärtigen Ende 40 des Behälters 24 und einem dem Isolator 16 zugewandten Ende 42 des Behälters 24. Durch die Erhöhung der Neigung der Auflagefläche 34 werden der Behälter 24 und somit die auch in dem Behälter 24 gespeicherten Verschlusselemente 12 in einen Zustand mit höherer potentieller Energie überführt.

Sobald ein Rutschwiderstand zwischen zueinander benachbarten Verschlusselementen 12 und/oder zwischen den Verschlusselementen 12 und einer Innenwandung des Behälters 24 überwunden ist, treten die Verschlusselemente 12 schwerkraftunterstützt aus dem Behälter 24 aus und gelangen zu einer im Innenraum 18 des Isolators angeordneten Verschlusselementleitung 44, die mit einem dem Behälter 24 zugewandten Überbrückungsabschnitt 58 innerhalb der Isolatoröffnung 20 angeordnet ist. Der Überbrückungsabschnitt 58 durchsetzt die Isolatoröffnung 20, sodass ein Kontakt der zu transportierenden Verschlusselemente 12 mit einer auch als "ring of concern" bezeichneten, potentiell nicht sterilen Berandung der Isolatoröffnung 20 vermieden wird.

Die Verschlusselementleitung 44 ist Teil einer insgesamt mit dem Bezugszeichen 46 bezeichneten Dosiereinrichtung. Diese umfasst optional weitere Bauteile, nämlich beispielsweise einen mit der Verschlusselementleitung 44 verbundenen Halter 48 und/oder eine Zähleinrichtung 50 zur Erfassung einer Anzahl von Verschlusselementen 12, welche die Verschlusselementleitung 44 passieren. Der Halter 48 dient zur Befestigung der Dosiereinrichtung 46 an einer (nicht dargestellten) isolatorfesten Aufnahme.

Die Verschlusselementleitung 44, der Halter 48 und der Zähler 50 können als Baugruppe 52 bereitgestellt sein, deren Handhabung entweder manuell oder aber vorzugsweise mit Hilfe eines Handhabungsabschnitts 54 der Baugruppe 52 erfolgt, der mit dem Wirkbereich eines Roboterarms koppelbar ist. Auf diese Weise kann die Dosiereinrichtung 46 bei Bedarf aus einem zur Isolatoröffnung 20 benachbarten Bereich entfernt werden, bspw. um die Isolatortür 22 schließen zu können. Eine solche Baugruppe 52 kann dann bei Bedarf aus dem Innenraum 18 des Isolators 16 ausgeschleust und gereinigt werden und anschließend wieder eingeschleust und wieder in dem zur Isolatoröffnung 20 benachbarten Bereich positioniert werden.

Die Verschlusselementleitung 44 weist einen Austrittsabschnitt 64 auf, der vorzugsweise oberhalb einer insgesamt mit dem Bezugszeichen 56 bezeichneten Sammeleinrichtung zum Sammeln der in den Innenraum 18 des Isolators transportierten Verschlusselemente 12 angeordnet ist. Bei der Sammeleinrichtung 56 handelt es sich im einfachsten Fall um eine schalenförmige Aufnahme, die vorzugsweise gerüttelt werden kann, sodass die Verschlusselemente 12 in einer Vorzugslage ausgerichtet und von einer vorzugsweise automatisierten (produzierenden) Handhabungseinrichtung ergriffen werden können, um in dem Innenraum 18 des Isolators 16 angeordnete Behältnisse in an sich bekannter Art und Weise verschließen zu können.

Der Aktor 38 ist vorzugsweise mit einer (nicht dargestellten) Steuereinheit verbunden, mittels welcher eine Soll-Menge von Verschlusselementen 12 vorgebbar ist und die einen Abgleich zu einer transportierten Ist-Menge von Verschlusselementen 12 ermöglicht, wobei diese Ist-Menge mittels der Zähleinrichtung 50 erfasst wird. Solange eine vorgegebene Soll-Menge kleiner als eine erfasste Ist-Menge von Verschlusselementen 12 ist, wird der Aktor 38 so angesteuert, dass Verschlusselemente 12 durch Erhöhung der Neigung α der Auflagefläche 34 in die Verschlusselementleitung 44 hinein gefördert werden. Ist die gewünschte Ist-Menge erreicht, wird die Neigung der Auflagefläche 40 nicht weiter erhöht oder verkleinert.

Zur Energiebeaufschlagung von in dem Behälter 24 angeordneten Verschlusselementen 12 ist es zusätzlich oder alternativ zu der vorzustehend unter Bezugnahme auf Fig. 1 beschriebenen Ausführungsform möglich, dass die Auflage 32 mit einem Aktor 38 zusammenwirkt, der den Vibrationszustand der Auflage 32 und somit auch der Auflagefläche 34 verändert, vergleiche Fig. 2. Diese Änderung des Vibrationszustands überträgt sich über den Behälter 24 auf die in dem Behälter 24 bevorrateten Verschlusselemente 12. Beispielsweise kann der Aktor 38 als Vibrationslinearförderer ausgebildet sein, der den Behälter 24 und somit auch die darin bevorrateten Verschlusselemente 12 in Vibration versetzt, wobei zumindest eine Bewegungskomponente der Vibration ausgehend von dem Behälter 24 in Richtung auf den Überbrückungsabschnitt 58 der Verschlusselementleitung 44 gerichtet ist.

Anstelle eines unter Bezugnahme auf Fig. 1 und 2 beschriebenen Behälters 24 in Form eines Beutels 26 kann auch ein Container 28 mit festen Behälterwänden verwendet werden, vergleiche Fig. 3. Auch ein solcher Behälter 24 kann mit Hilfe eines Aktors 38 in einen Zustand mit größerer Neigung und/oder in einen Vibrationszustand versetzt werden.

Es ist bevorzugt, dass der Container 28 eine ringförmige Membran 60 aufweist, die einen flexiblen Übergang zwischen einer Berandung 62 des Containers 28 und einer Außenwand des Isolators 16 bereitstellt und die gleichzeitig gewährleistet, dass ein Innenraum des Containers 28 von der Umgebung 14 des Isolators 16 entkoppelt bleibt und darin bevorratete Verschlusselemente 12 nicht kontaminiert werden.

Erfindungsgemäß ist vorgesehen, dass entlang des Transportweges von der Isolatoröffnung 20 zu der Sammeleinrichtung 56 eine Fördereinheit 66 verwendet wird. Solche Fördereinheiten werden unter Bezugnahme auf die nachfolgenden Zeichnungen beschrieben. Diese Fördereinheiten 66 sind alternativ oder zusätzlich zu einem unter Bezugnahme auf Fig. 1 bis 3 beschriebenen Aktor 38 vorgesehen.

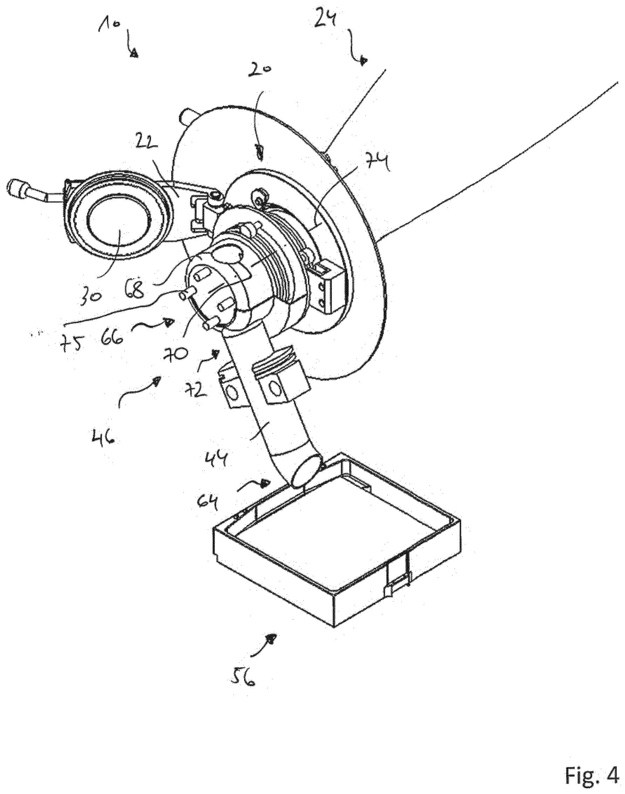

Eine Fördereinheit 66 des Ausführungsbeispiels gemäß Fig. 4 bis 6 umfasst eine Fördertrommel 68 mit einer Trommelöffnung 70, welche in Fluchtlage mit einem Eingangsabschnitt 72 einer der Fördereinheit 66 nachgeschalteten Verschlusselementleitung 44 bringbar ist. Zu diesem Zweck ist die Fördertrommel 68 um eine zentrale Trommelachse 74 drehbar antreibbar. Hierfür ist eine (nicht dargestellte) Förderantriebseinrichtung, beispielsweise ein Motor, vorgesehen, der unmittelbar oder über Getriebeelemente mindestens ein Antriebselement 75 der Fördertrommel 68 antreibt. Solche Antriebselemente 75 können beispielsweise zapfenförmig ausgebildet sein.

Der Querschnitt der Trommelöffnung 70 ist größer als ein maximaler Querschnitt eines Verschlusselements 12 und vorzugsweise kleiner als ein maximaler Querschnitt von zwei in Transportrichtung gesehen auf identischer Höhe angeordneten Verschlusselementen 12.

Innerhalb der Fördertrommel 68 und fest mit der Fördertrommel 68 verbunden sind Förderelemente 76, 78 angeordnet, welche sich winklig, insbesondere im Wesentlichen senkrecht, zu einer Transportachse 80 der Verschlusselemente 12 erstrecken. Die Förderelemente 76, 78 können beispielsweise als Leitstege oder -bleche ausgebildet sein. Entlang der Transportachse 80 gesehen begrenzen die Förderelemente 76 und 78 eine Förderkammer 82, die nach außen hin durch eine Innenwandung der Fördertrommel 68 begrenzt ist. Die Förderkammer 82 dient zur vereinzelten Förderung von Verschlusselementen 12 in die Verschlusselementleitung 44 hinein.

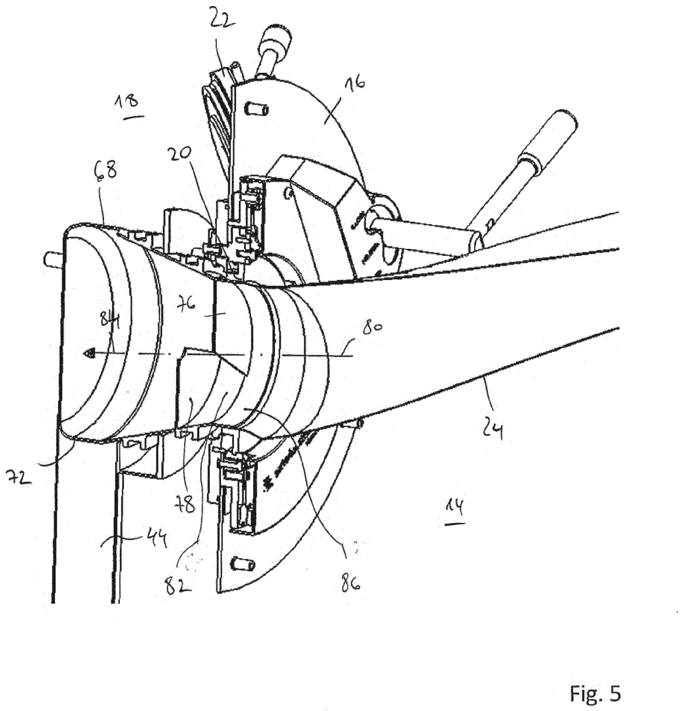

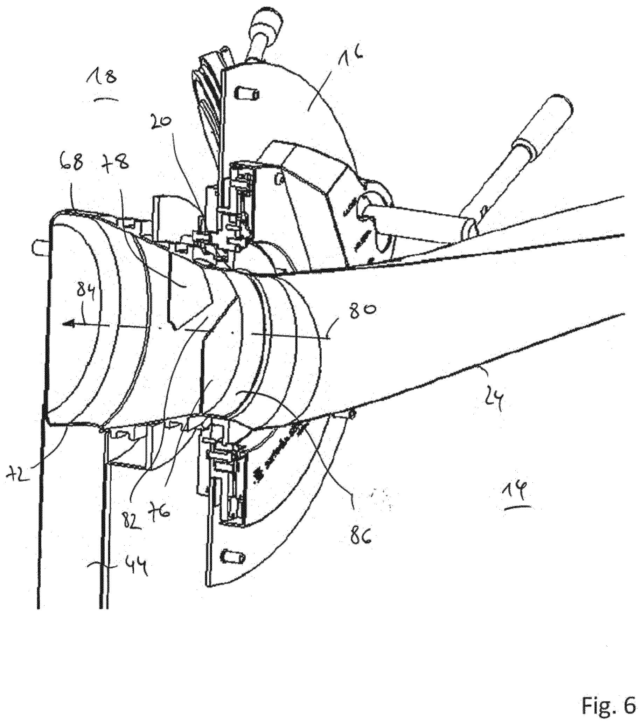

In den Fig. 5 und 6 sind jeweils unterschiedliche, einander ergänzende Abschnitte der Förderelemente 76 und 78 dargestellt, wobei die Fördertrommel 68 in den Fig. 5 und 6 relativ zueinander um 180° verdrehte Lagen um die Trommelachse 74 einnimmt.

Aus dem Behälter 24 entlang der Transportachse 80 zu transportierende Verschlusselemente 12 folgen einer in Fig. 5 und 6 mit dem Bezugszeichen 84 bezeichneten Transportrichtung. Ausgehend von dem Behälter 24 und in Transportrichtung 84 gesehen, weist die Fördertrommel 68 einen Fördertrommelabschnitt 86 auf. Der Fördertrommelabschnitt 86 überbrückt die Türöffnung 20 zwischen dem in der Umgebung 14 angeordneten Behälter 24 und dem Innenraum 18 des Isolators 16.

Die Fördertrommel 68 weist im Bereich der Förderkammer 82 in Transportrichtung 84 gesehen einen sich hohlkegelförmig erweiternden Innenraum auf, vergleiche Fig. 5 und 6.

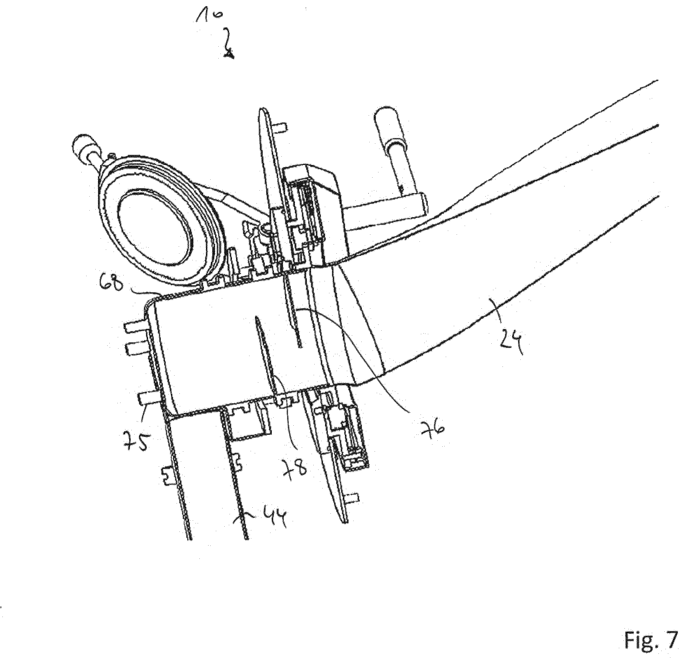

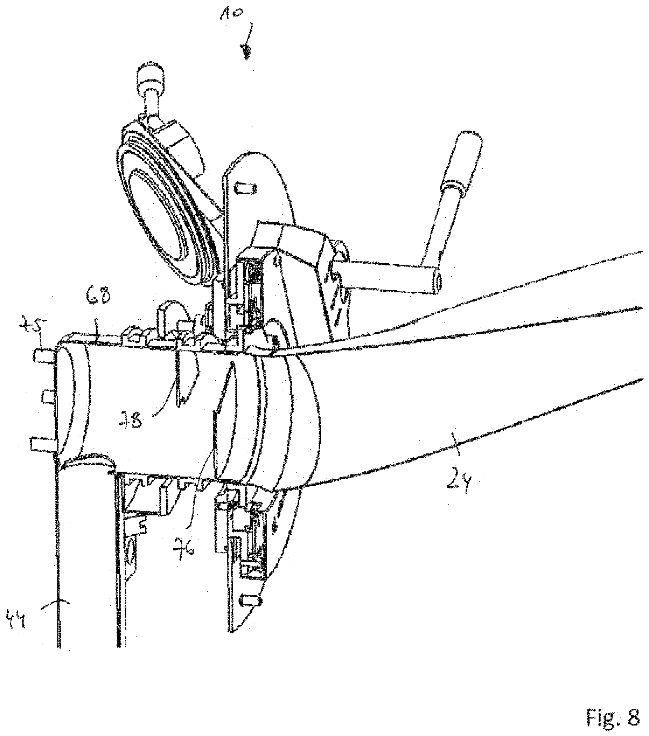

Alternativ zu einer sich hohlkegelförmig erweiternden Fördertrommel 68 kann auch eine zylindrische Trommel 68 verwendet werden, vergleiche Fig. 7 und 8. Im Übrigen wird für das Ausführungsbeispiel gemäß Fig. 7 und 8 auf die vorstehende Beschreibung des Ausführungsbeispiels zu Fig. 4 bis 6 Bezug genommen.

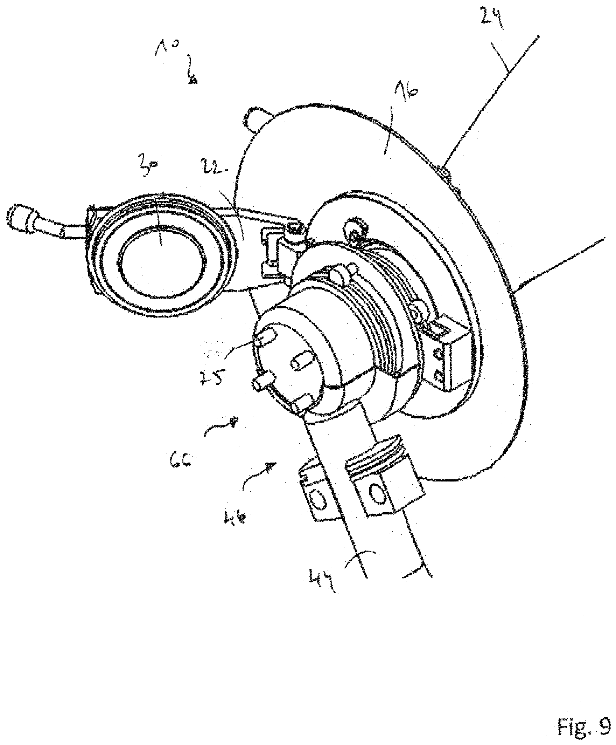

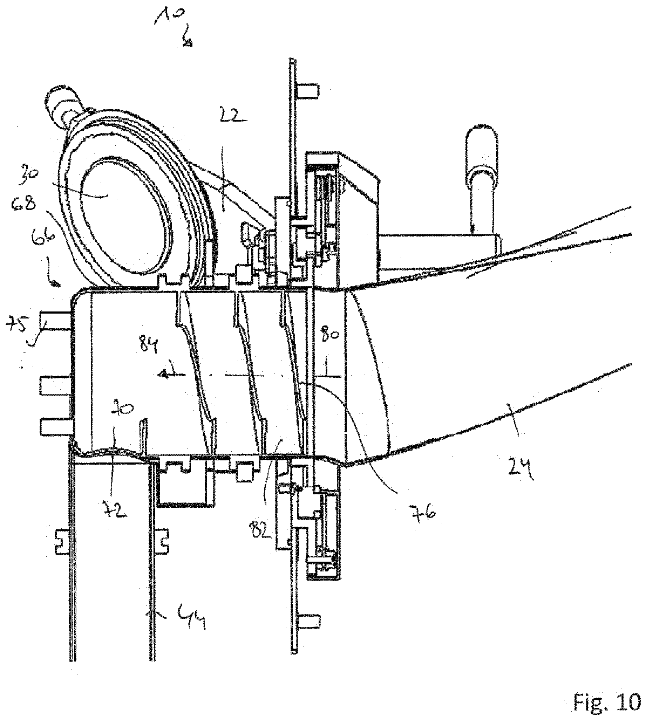

Ein in den Fig. 9 und 10 dargestelltes Ausführungsbeispiel unterscheidet sich von den vorstehend unter Bezugnahme auf Fig. 4 bis 8 dargestellten Ausführungsformen dadurch, dass ein Förderelement 76 in Form einer Förderschnecke vorgesehen ist. Das Förderelement 76 ist drehfest mit der Fördertrommel 68 verbunden, die insbesondere zylindrisch ausgebildet ist. Vorzugsweise nimmt die Steigung des schneckenförmigen Förderelements 76 in Transportrichtung 84 der Verschlusselemente 12 gesehen zu, sodass sich ein Volumen der Förderkammer 82 in Transportrichtung 84 gesehen vergrößert.

Bei den Ausführungsbeispielen gemäß Fig. 4 bis 10 treibt die (nicht dargestellte) Förderantriebseinrichtung den mindestens einen Antriebsabschnitt 75 der Fördereinheit 66 an, sodass sowohl die Fördertrommel 68 als auch die in der Fördertrommel 68 angeordneten Förderelemente 76, 78 in Drehung um die Trommelachse 74 versetzt werden.

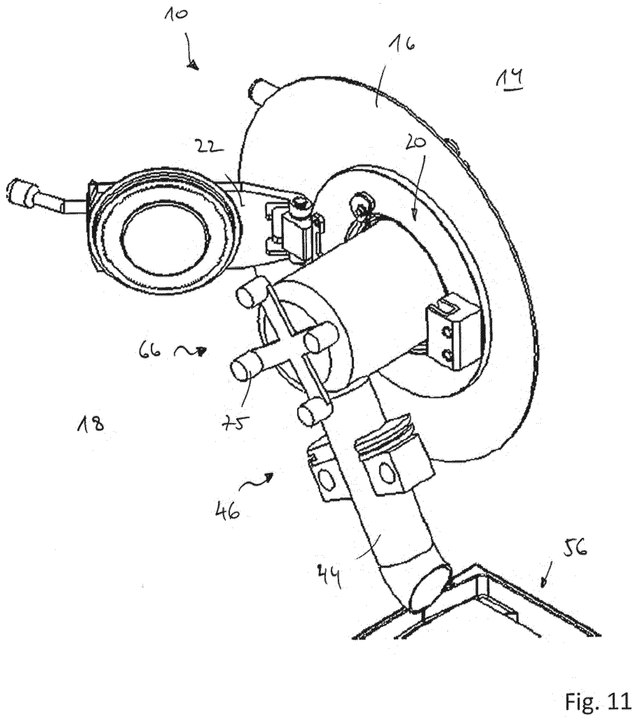

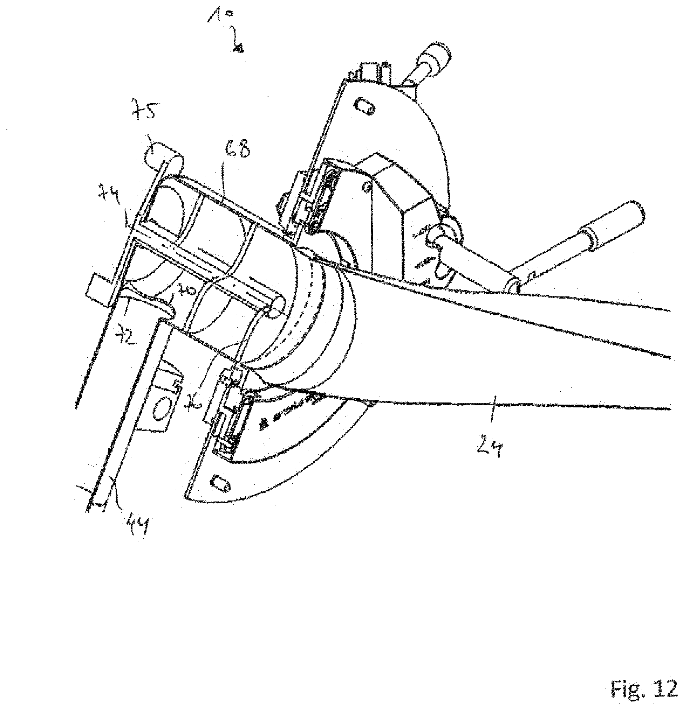

Als Alternative kann auch eine feststehende Fördertrommel 68 verwendet werden, vergleiche Fig. 11 und 12, wobei der mindestens eine Antriebsabschnitt 75 lediglich mit mindestens einem Förderelement 76 zusammenwirkt und nicht mit der Fördertrommel 68. Mit anderen Worten: Mindestens ein Förderelement 76 wird um eine feststehende Trommelachse 74 einer feststehenden Fördertrommel 68 in Drehung versetzt. Eine solche Anordnung eignet sich insbesondere für Förderelemente 76 in Form einer Förderschnecke oder - spirale.

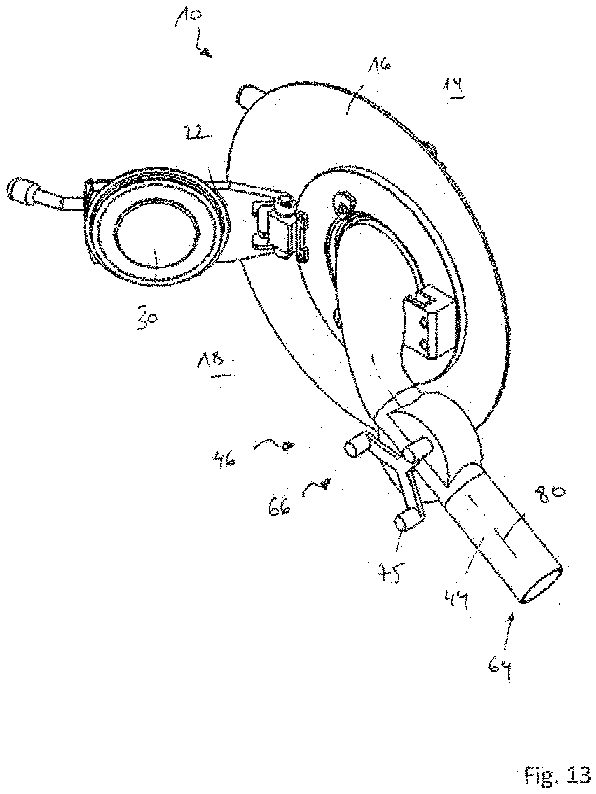

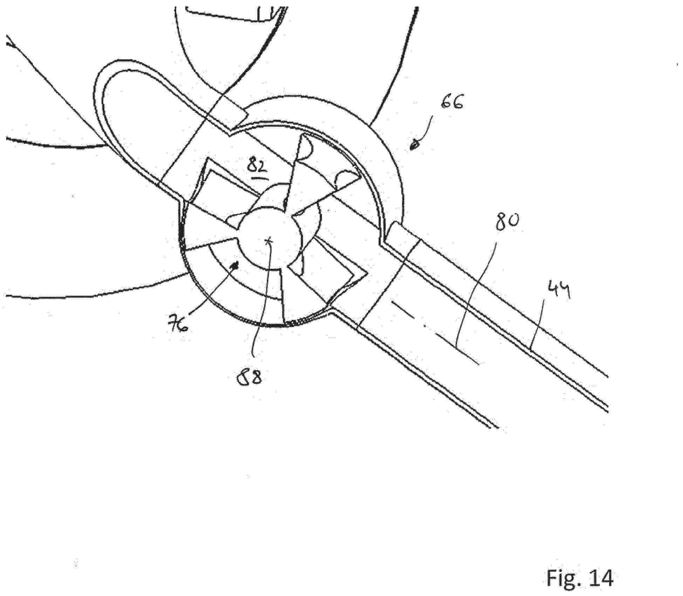

Bei den vorstehend unter Bezugnahme auf Fig. 4 bis 12 beschriebenen Ausführungsbeispielen verläuft eine Drehachse der Fördereinheit 66 zumindest im Wesentlichen parallel zu einer Transportachse 80 der Verschlusselemente 12. Davon abweichend beziehen sich die Fig. 13 und 14 auf ein Ausführungsbeispiel eines Systems 10 mit einer Fördereinheit 66, deren Drehachse 88 senkrecht zu einer Transportachse 80 der Verschlusselemente 12 verläuft. Das Förderelement 76 der Fördereinheit 66 des Systems 10 gemäß Fig. 13 und 14 ist als Förderrad ausgebildet, das in vorstehend beschriebener Art und Weise über mindestens einen Antriebsabschnitt 75 drehbar antreibbar ist.

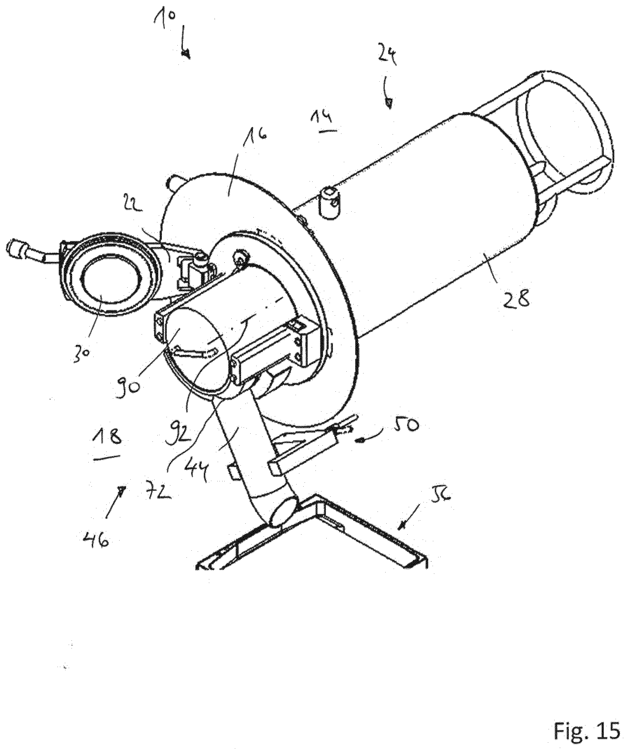

In Fig. 15 ist ein System 10 dargestellt, das sich für Behälter 24 in Form eines Containers 28 eignet. Der Container 28 weist einen Auszug 90 auf, der bei Anordnung des Behälters 24 an einem Isolator 16 aus der Umgebung 14 des Isolators 16 in den Innenraum 18 des Isolators 16 hinein verlagerbar ist. Der Auszug 90 ist außerdem mittels einer (nicht dargestellten) Behälterantriebseinrichtung um eine zentrale Behälterachse 92 drehbar. Der Auszug 90 weist eine (nicht dargestellte) Öffnung auf, welche einer Öffnung 70 einer Fördertrommel 68 entspricht, vergleiche beispielsweise Fig. 12. Diese Öffnung kann mit einem Eingangsabschnitt 72 einer Verschlusselementleitung 44 in Fluchtlage gebracht werden, wodurch Verschlusselemente 12 aus dem Behälter 24 über den Innenraum des Auszugs 90 in die Verschlusselementleitung 44 gelangen und einer Sammeleinrichtung 56 zugeführt werden.

## Patentansprüche

1. System (10) zum Transport von sterilen schüttfähigen Verschlusselementen (12) aus einer Umgebung (14) eines Isolators (16) in einen Innenraum (18) des Isolators (16), umfassend einen Behälter (24) zur Speicherung einer Vorrats-Menge der Verschlusselemente (12) in der Umgebung (14) des Isolators (16), wobei der Behälter (24) als Beutel (26) oder als Container (28) ausgebildet ist und zum Transport der sterilen Verschlusselemente (12) dient, eine Isolatoröffnung (20) mit einer Isolatortür (22) zum Verschließen der Isolatoröffnung (20), eine Sammeleinrichtung (56) zum Sammeln der Verschlusselemente (12) und zur Bereitstellung der Verschlusselemente (12) in dem Innenraum (18) des Isolators (16), eine Dosiervorrichtung (46) zur Steuerung einer aus dem Behälter (24) durch die Isolatoröffnung (20) hindurch und in die Sammeleinrichtung (56) zu transportierenden Soll-Menge von Verschlusselementen (12), **dadurch gekennzeichnet, dass** die Dosiervorrichtung (46) mindestens eine Fördereinheit (66) umfasst, mit einer Förderkammer (82), deren Volumen größer ist als das Volumen eines einzelnen Verschlusselements (12) und kleiner als das Volumen von zwei Verschlusselementen (12), wobei die Fördereinheit (66) entlang des Transportweges von der Isolatoröffnung (20) zu der Sammeleinrichtung (56) verwendet wird.

2. System (10) nach Anspruch 1, **dadurch gekennzeichnet, dass** die Dosiervorrichtung (46) mindestens einen Aktor (38) aufweist, der in Abhängigkeit der zu transportierenden Soll-Menge zwischen einem Ruhezustand und einem Gebrauchszustand schaltbar ist.

3. System (10) nach Anspruch 2, **dadurch gekennzeichnet, dass** der Gebrauchszustand mit einer Erhöhung der potentiellen und/oder kinetischen Energie zumindest einer Teilmenge der Vorrats-Menge der Verschlusselemente (12) einhergeht.

4. System (10) nach Anspruch 2 oder 3, **dadurch gekennzeichnet, dass** eine in der Umgebung (14) des Isolators (16) angeordnete Auflage (32) für den Behälter (24) vorgesehen ist, wobei der mindestens eine Aktor (38) die Neigung und/oder einen Vibrationszustand der Auflage (32) und des Behälters (24) verändert.

5. System (10) nach einem der vorstehenden Ansprüche, **dadurch gekennzeichnet, dass** die Fördereinheit (66) eine Fördertrommel (68) aufweist, welche eine - bezogen auf eine Transportachse (80) der Verschlusselemente (12) - äußere Begrenzung der Förderkammer (82) bildet.

6. System (10) nach Anspruch 5, **dadurch gekennzeichnet,**
**dass** die Fördertrommel (68) bezogen auf eine Transportachse (80) der Verschlusselemente (12) einen hohlzylindrischen Abschnitt aufweist und/oder sich in Transportrichtung (84) der Verschlusselemente (12) gesehen hohlkegelförmig erweitert.

7. System (10) nach einem der Ansprüche 5 bis 6 , **dadurch gekennzeichnet, dass** die Fördereinheit (66) mindestens ein Förderelement (76, 78) aufweist, das die Förderkammer (82) entlang einer Transportachse (80) der Verschlusselemente (12) gesehen begrenzt.

8. System (10) nach einem der Ansprüche 5 bis 7 **dadurch gekennzeichnet, dass** die Dosiervorrichtung (46) eine in Abhängigkeit der zu transportierenden Soll-Menge schaltbare Förderantriebseinrichtung zum Drehantrieb der Fördertrommel (68) und/oder der Förderelemente (76, 78) umfasst.

9. System (10) nach einem der vorstehenden Ansprüche, **dadurch gekennzeichnet, dass** die Dosiereinrichtung (46) eine Behälterantriebseinrichtung zur Drehung des Behälters (24) oder eines Teilabschnitts des Behälters (24) um eine zentrale Behälterachse umfasst.

10. System (10) nach einem der vorstehenden Ansprüche, **dadurch gekennzeichnet, dass** die Dosiereinrichtung (46) eine Verschlusselementleitung (44) umfasst, welche einen Überbrückungsabschnitt (58) aufweist, der innerhalb der Isolatoröffnung (20) angeordnet ist.

11. System (10) nach einem der vorstehenden Ansprüche, **dadurch gekennzeichnet, dass** die Dosiereinrichtung (46) eine Zähleinrichtung (50) zur Erfassung einer Anzahl, eines Gewichts und/oder eines Volumens der Verschlusselemente (12) umfasst, wobei vorzugsweise die Zähleinrichtung (50) in Transportrichtung der Verschlusselemente (12) gesehen vor der Sammeleinrichtung (56) angeordnet ist.

12. System (10) nach einem der vorstehenden Ansprüche, **dadurch gekennzeichnet, dass** die Dosiervorrichtung (46) oder Teile der Dosiervorrichtung (46) als innerhalb des Isolators (16) bewegbare Baugruppe (52) bereitgestellt ist oder sind, welche vorzugsweise mittels eines Roboterarms positionierbar ist.

13. System (10) nach einem der vorstehenden Ansprüche, **dadurch gekennzeichnet, dass** die Sammeleinrichtung (56) als Sortiereinrichtung ausgebildet ist, wobei vorzugsweise der Sortiereinrichtung innerhalb des Isolators (16) kein Zwischenspeicher für Verschlusselemente (12) vorgeschaltet ist.

## Claims

1. System (10) for transporting loose sterile closure elements (12) from an environment (14) of an isolator (16) into an interior (18) of the isolator (16), comprising a receptacle (24) for storing a stock quantity of the closure elements (12) in the environment (14) of the isolator (16), the receptacle (24) being designed as a bag (26) or as a container (28) and serving for transporting the sterile closure elements (12), an isolator opening (20) which has an isolator door (22) for closing the isolator opening (20), a collecting means (56) for collecting the closure elements (12) and for providing the closure elements (12) in the interior (18) of the isolator (16), a metering device (46) for controlling a target quantity of closure elements (12) to be transported from the receptacle (24) through the isolator opening (20) and into the collecting means (56), **characterized in that** the metering device (46) comprises at least one conveyor unit (66) having a conveyor chamber (82), the volume of which is greater than the volume of a single closure element (12) and smaller than the volume of two closure elements (12), the conveyor unit (66) being used along the transport path from the isolator opening (20) to the collecting means (56).

2. System (10) according to claim 1, **characterized in that** the metering device (46) has at least one actuator (38) which can be switched between a rest state and a use state depending on the target quantity to be transported.

3. System (10) according to claim 2, **characterized in that** the use state is associated with an increase in the potential and/or kinetic energy of at least a portion of the stock quantity of the closure elements (12).

4. System (10) according to claim 2 or 3, **characterized in that** a support (32) arranged in the environment (14) of the isolator (16) is provided for the receptacle (24), the at least one actuator (38) changing the inclination and/or a vibration state of the support (32) and the receptacle (24).

5. System (10) according to any of the preceding claims,
**characterized in that** the conveyor unit (66) has a conveyor drum (68) which forms an outer boundary of the conveyor chamber (82) with respect to a transport axis (80) of the closure elements (12).

6. System (10) according to claim 5, **characterized in that** the conveyor drum (68) has a hollow cylindrical portion relative to a transport axis (80) of the closure elements (12) and/or widens in the shape of a hollow cone as seen in the transport direction (84) of the closure elements (12).

7. System (10) according to one of claims 5 to 6, **characterized in that** the conveyor unit (66) has at least one conveyor element (76, 78) which delimits the conveyor chamber (82) as seen along a transport axis (80) of the closure elements (12).

8. System (10) according to any of claims 5 to 7, **characterized in that** the metering device (46) comprises a conveyor drive means which can be switched depending on the target quantity to be transported and which is used to drive the conveyor drum (68) and/or the conveyor elements (76, 78) in rotation.

9. System (10) according to any of the preceding claims, **characterized in that** the metering device (46) comprises a receptacle drive means for rotating the receptacle (24) or a portion of the receptacle (24) about a central receptacle axis.

10. System (10) according to any of the preceding claims,
**characterized in that** the metering device (46) comprises a closure element line (44) which has a bridging portion (58) arranged within the isolator opening (20).

11. System (10) according to any of the preceding claims,
**characterized in that** the metering device (46) comprises a counting means (50) for detecting a number, a weight and/or a volume of the closure elements (12), the counting means (50) preferably being arranged upstream of the collecting means (56) as seen in the transport direction of the closure elements (12).

12. System (10) according to any of the preceding claims,
**characterized in that** the metering device (46) or parts of the metering device (46) is or are provided as an assembly (52) which is movable within the isolator (16) and which is preferably positionable by means of a robot arm.

13. System (10) according to any of the preceding claims,
**characterized in that** the collecting means (56) is designed as a sorting means, preferably no intermediate storage means for closure elements (12) being arranged upstream of the sorting means within the isolator (16).

## Revendications

1. Système (10) pour le transport d'éléments de fermeture (12) stériles en vrac hors d'un environnement (14) d'un isolateur (16) dans un espace intérieur (18) de l'isolateur (16), comprenant un récipient (24) pour le stockage d'une quantité de réserve des éléments de fermeture (12) dans l'environnement (14) de l'isolateur (16), dans lequel le récipient (24) est réalisé sous forme de sac (26) ou sous forme de conteneur (28) et sert au transport des éléments de fermeture (12) stériles, une ouverture d'isolateur (20) comportant une porte d'isolateur (22) pour la fermeture de l'ouverture d'isolateur (20), un appareil de collecte (56) pour la collecte des éléments de fermeture (12) et pour la fourniture des éléments de fermeture (12) dans l'espace intérieur (18) de l'isolateur (16), un dispositif de dosage (46) pour la commande d'une quantité de consigne d'éléments de fermeture (12) à transporter hors du récipient (24) à travers l'ouverture d'isolateur (20) et dans l'appareil de collecte (56),
**caractérisé en ce que** le dispositif de dosage (46) comprend au moins une unité d'acheminement (66) comportant une chambre d'acheminement (82) dont le volume est supérieur au volume d'un seul élément de fermeture (12) et inférieur au volume de deux éléments de fermeture (12), dans lequel l'unité d'acheminement (66) est utilisée le long du trajet de transport de l'ouverture d'isolateur (20) vers l'appareil de collecte (56).

2. Système (10) selon la revendication 1, **caractérisé en ce que** le dispositif de dosage (46) présente au moins un actionneur (38) qui peut être commuté entre un état de repos et un état d'utilisation en fonction de la quantité de consigne à transporter.

3. Système (10) selon la revendication 2, **caractérisé en ce que** l'état d'utilisation s'accompagne d'une augmentation de l'énergie potentielle et/ou cinétique d'au moins une quantité partielle de la quantité de réserve des éléments de fermeture (12).

4. Système (10) selon la revendication 2 ou 3, **caractérisé en ce qu'un** support (32) disposé dans l'environnement (14) de l'isolateur (16) est prévu pour le récipient (24), dans lequel l'au moins un actionneur (38) modifie l'inclinaison et/ou un état de vibration du support (32) et du récipient (24).

5. Système (10) selon l'une des revendications précédentes,
**caractérisé en ce que** l'unité d'acheminement (66) présente un tambour d'acheminement (68) qui forme une limite extérieure, par rapport à un axe de transport (80) des éléments de fermeture (12), de la chambre d'acheminement (82).

6. Système (10) selon la revendication 5, **caractérisé en ce que** le tambour d'acheminement (68) présente une section cylindrique creuse par rapport à un axe de transport (80) des éléments de fermeture (12) et/ou s'élargit en forme de cône creux, vu dans la direction de transport (84) des éléments de fermeture (12).

7. Système (10) selon l'une des revendications 5 à 6, **caractérisé en ce que** l'unité d'acheminement (66) présente au moins un élément d'acheminement (76, 78) qui délimite la chambre d'acheminement (82), vu le long d'un axe de transport (80) des éléments de fermeture (12).

8. Système (10) selon l'une des revendications 5 à 7, **caractérisé en ce que** le dispositif de dosage (46) comprend un appareil d'entraînement d'acheminement, commutable en fonction de la quantité de consigne à transporter, pour l'entraînement en rotation du tambour d'acheminement (68) et/ou des éléments d'acheminement (76, 78).

9. Système (10) selon l'une des revendications précédentes,
**caractérisé en ce que** l'appareil de dosage (46) comprend un appareil d'entraînement de récipient pour la rotation du récipient (24) ou d'une section partielle du récipient (24) autour d'un axe central de récipient.

10. Système (10) selon l'une des revendications précédentes,
**caractérisé en ce que** l'appareil de dosage (46) comprend une conduite d'élément de fermeture (44) qui présente une section de pontage (58) qui est disposée à l'intérieur de l'ouverture d'isolateur (20).

11. Système (10) selon l'une des revendications précédentes,
**caractérisé en ce que** l'appareil de dosage (46) comprend un appareil de comptage (50) pour la détection d'un nombre, d'un poids et/ou d'un volume des éléments de fermeture (12), dans lequel l'appareil de comptage (50) est de préférence disposé avant l'appareil de collecte (56), vu dans la direction de transport des éléments de fermeture (12).

12. Système (10) selon l'une des revendications précédentes,
**caractérisé en ce que** le dispositif de dosage (46) ou des parties du dispositif de dosage (46) sont fournis sous forme d'ensemble (52) mobile à l'intérieur de l'isolateur (16), lequel ensemble peut être positionné de préférence au moyen d'un bras de robot.

13. Système (10) selon l'une des revendications précédentes,
**caractérisé en ce que** l'appareil de collecte (56) réalisé sous forme d'appareil de tri, dans lequel de préférence aucun stockage intermédiaire pour éléments de fermeture (12) n'est monté en amont de l'appareil de tri à l'intérieur de l'isolateur (16).
